# NEUE EUROPÄISCHE PATENTSCHRIFT

(11) **EP 1 474 102 B2**
(45) Veröffentlichungstag und Bekanntmachung des Hinweises auf die Entscheidung über den Einspruch: **21.01.2015**
(45) Hinweis auf die Patenterteilung: 25.04.2012
(21) Anmeldenummer: 02796692.8
(22) Anmeldetag: 20.12.2002
(51) Int. Cl.: A61K 8/39, A61K 8/60, A61K 8/86, A61Q 5/12

(54) **Verdickungsmittel für tensidhaltige Formulierungen**
Thickeners for use in surfactant-containing formulations
Epaississant destiné à des formulations contenant des tensio-actifs

(30) Priorität: 01.02.2002 DE 10204099
(43) Veröffentlichungstag der Anmeldung: 10.11.2004
(73) Patentinhaber: Beiersdorf AG, 20245 Hamburg (DE)
(72) Erfinder: ARGEMBEAUX, Horst, 21465 Wentorf (DE); HEITMANN, Birgit, 22769 Hamburg (DE)
(86) Internationale Anmeldenummer: PCT/EP2002/014611
(87) Internationale Veröffentlichungsnummer: WO 2003/063818

(56) Entgegenhaltungen:
- EP-A- 1 055 407
- WO-A-03/013459
- WO-A-03/013467
- WO-A1-94/16676
- WO-A1-99/63032
- DE-A1- 19 818 410
- US-A- 5 807 561
- DATABASE CAPLUS [Online] CHEMICAL ABSTRACTS SERVICE, COLUMBUS, OHIO, US; Accession-Number 1983:489879, XP002237981 & POLOVSKY, STUART B. ET AL.: "Comunicationes presentadas a la jornadas del comite Espanol de la detergencia, 14th. Ed., 1983 "Peg-120 methyl glucoside dioleate." 1983 , EDISON , NJ, 08818 USA Seite 83 -Seite 102

## Beschreibung

Die Erfindung betrifft kosmetische oder dermatologische Zubereitungen umfassend eine Kombination aus, Verdickungsmittel und deren Verwendung. Die synergistische Kombination der Verdickungsmittel führt zu kosmetischen Zubereitungen, insbesondere Zubereitungen mit einem hohen nichtionischen Tensidanteil, mit äußerst positiven Eigenschaften. Die Zubereitungen, insbesondere Haarreinigungs- und pflegezubereitungen, weisen eine vorteilhafte Viskosität auf, sind ästhetisch und lassen sich gut auf Haut und Haaren verteilen.

Zum Verdicken tensidhaltiger Formulierungen sind dem Fachmann viele Stoffe, wie aus der Fachliteratur, z.B. K.Schrader, "Grundlagen und Rezepturen der Kosmetik", 2.Auflage,1989, Hüthig Buchverlag, Heidelberg, bekannt.

Prinzipiell können zwei Arten der Verdickung unterschieden werden:
Beeinflussung der Micellstruktur der eingesetzten Tenside durch den Einsatz von Elektrolyten und/oder amphiphilen Substanzen oder der Einsatz von Hydrokolloiden. Erste Methode beschränkt sich jedoch auf wenige, vornehmlich anionische und amphotere Tenside und erlaubt auch nur einen sehr begrenzten Einfluss auf die Viskosität tensidhaltiger Zubereitungen.
"Hydrokolloid" ist die technologische Kurzbezeichnung für die an sich richtigere Bezeichnung "hydrophiles Kolloid". Hydrokolloide sind Makromoleküle, die eine weitgehend lineare Gestalt haben und über intermolekulare Wechselwirkungskräfte verfügen, die Neben- und Hauptvalenzbindungen zwischen den einzelnen Molekülen und damit die Ausbildung eines netzartigen Gebildes ermöglichen. Sie sind teilweise wasserlösliche natürliche oder synthetische Polymere, die in wässrigen Systemen Gele oder viskose Lösungen bilden. Sie erhöhen die Viskosität des Wassers, indem sie entweder Wassermoleküle binden (Hydratation) oder aber das Wasser in ihre unter sich verflochtenen Makromoleküle aufnehmen und einhüllen, wobei sie gleichzeitig die Beweglichkeit des Wassers einschränken. Solche wasserlöslichen Polymere stellen eine große Gruppe chemisch sehr unterschiedlicher natürlicher und synthetischer Polymere dar, deren gemeinsames Merkmal ihre Löslichkeit in Wasser bzw. wässrigen Medien ist. Voraussetzung dafür ist, dass diese Polymere über eine für die Wasserlöslichkeit ausreichende Anzahl an hydrophilen Gruppen besitzen und nicht zu stark vernetzt sind. Die hydrophilen Gruppen können nichtionischer, anionischer oder kationischer Natur sein, beispielsweise wie folgt:

| | | | | |
|---|---|---|---|---|
| -NH₂ | -COOH | -COO⁻ | M⁺ | 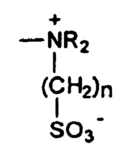 |
| -NH-R | 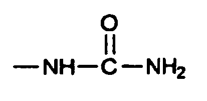 | -SO₃⁻ | M⁺ | |
| -OH | | 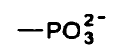 | M²⁺ | |
| -SH | 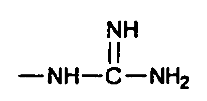 | 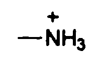 | X⁻ | 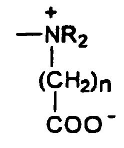 |
| -O- | | 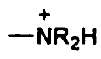 | X⁻ | |
| 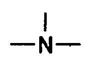 | 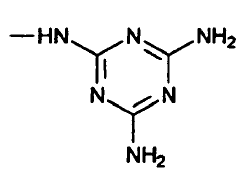 | 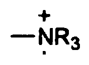 | X⁻ | |
| | | 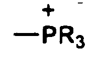 | X⁻ | |
| | | 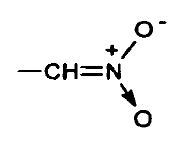 | | |

Die Gruppe der kosmetisch und dermatologisch relevanten Hydrokolloide lässt sich wie folgt einteilen in:
a. Aus der Natur stammende Polymere, die als Verdickungsmittel Verwendung finden, sind beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine und Casein.
b. Abgewandelte Naturstoffe stammen vor allem aus der Gruppe der modifizierten Stärken und Cellulosen, beispielhaft seien hier Carboxymethylcellulose und andere Celluloseether, Hydroxyethyl- und -propylcellulose sowie Kernmehlether genannt.
c. Eine grosse Gruppe von Verdickungsmitteln, die breite Verwendung in den unterschiedlichsten Anwendungsgebieten finden, sind vollsynthetische Polymere wie Polyacryl- und Polymethacryl-Verbindungen, Vinylpolymere, Polycarbonsäuren, Polyether, Polyimine, Polyamide und Polyurethane und/oder anorganische Verbindungen, wie z. B. Polykieselsäuren, Tonmineralien wie Montmorillonite, Zeolithe, Kieselsäuren.

Nachteil des Einsatzes von Hydrokolloiden ist häufig eine mangelhafte Elektrolytverträglichkeit und ein pH-Wert abhängiges Wirkspektrum. Kosmetische Reinigungsformulierungen enthalten vielfach als Nebenbestandteile Elektrolyte. Außerdem ist für kosmetische Formulierungen häufig ein leicht saurer pH-Wert von ca. 5 vorteilhaft, da dieser dem pH-Wert der Haut entspricht und somit zu einer guten Verträglichkeit beiträgt. Darüber hinaus lässt ein solcher pH-Wert den Einsatz besonders milder Konservierungsmittel, wie z.B. Benzoesäure und deren Salze, zu, deren Wirkspektrum bei höheren pH-Werten abnimmt.

Eine Aufgabe der vorliegenden Erfindung ist es daher Verdickungsmittel bereit zu stellen, die diese Nachteile des Standes der Technik nicht aufweisen.

Als weitere Verdickungsmittel (Vernetzer) sind Verbindungen bekannt, die aus folgenden Gruppen ausgewählt werden können.
- Polyethylenglycolether der allgemeinen Formel R-O-(-CH₂-CH₂-O-)ₙ-R', wobei R und R' unabhängig voneinander verzweigte oder unverzweigte Alkyl-, Aryl- oder Alkenylreste und n eine Zahl größer als 100 darstellen,
- veretherten Fettsäureethoxylate der allgemeinen Formel R-COO-(-CH₂-CH₂-O-)ₙ -R', wobei R und R' unabhängig voneinander verzweigte oder unverzweigte Alkyl-, Aryl- oder Alkenylreste und n eine Zahl größer als 100 darstellen,
- veresterten Fettsäureethoxylate der allgemeinen Formel R-COO-(-CH₂-CH₂-O-)ₙ -C(O)-R', wobei R und R' unabhängig voneinander verzweigte oder unverzweigte Alkyl-, Aryl- oder Alkenylreste und n eine Zahl größer als 100 darstellen, der Polypropylenglycolether der allgemeinen Formel R-O-(-CH₂-CH(CH₃)-O-)ₙ-R', wobei R und R' unabhängig voneinander verzweigte oder unverzweigte Alkyl-, Aryl- oder Alkenylreste und n eine Zahl größer als 100 darstellen, der veresterten Fettsäurepropoxylate der allgemeinen Formel R-COO-(-CH₂-CH(CH₃)-O-)ₙ-C(O)-R', wobei R und R' unabhängig voneinander verzweigte oder unverzweigte Alkyl-, Aryl- oder Alkenylreste und n eine Zahl größer als 100 darstellen, der Polypropylenglycolether der allgemeinen Formel R-O-Xₙ-Yₘ-R', wobei R und R' unabhängig voneinander verzweigte oder unverzweigte Alkyl-, Aryl- oder Alkenylreste darstellen, wobei X und Y nicht identisch sind und jeweils entweder eine Oxyethylengruppe oder eine Oxypropylengruppe und n und m unabhängig voneinander Zahlen darstellen, deren Summe größer als 100 ist
- veretherten Fettsäurepropoxylate der allgemeinen Formel R-COO-Xₙ-Yₘ-R', wobei R und R' unabhängig voneinander verzweigte oder unverzweigte Alkyl-, Aryl- oder Alkenylreste darstellen, wobei X und Y nicht identisch sind und jeweils entweder eine Oxyethylengruppe oder eine Oxypropylengruppe und n und m unabhängig voneinander Zahlen darstellen, deren Summe größer als 100 ist

Bekannt sind insbesondere das PEG-150-Distearat, PEG 800 Distearat, PEG-800 Chol₂, das PEG-150-Dioleat, das PEG-300-Pentaerythrityltetraisostearat, das PEG-120 Methylglucosedioleat, das PEG-160-Sorbitantriisostearat, das PEG-450-Sorbitolhexaisostearat, das PEG-230-Glyceryltriisostearat, das PEG-200 Hydrogenated Glyceryl Palmitat, der Verdicker der Firma Südchemie mit der Bezeichnung Purethix 1442 (Polyether-1), Polyurethan-Verdicker, wie Rheolate 204, 205, 208 (Firma Rheox) oder davon abgewandelte kosmetische Varianten bzw. DW 1206B von Rhom & Haas oder Serad Fx 1010, 1035 von Hüls.

Hydrophob modifizierte, synthetische oder natürliche Polymere werden gelegentlich auch als assoziative Verdicker bezeichnet. Dazu zählen hydrophob substituierte Polysaccharidderivate, beispielsweise hydrophob substituierte Celluloseether, hydrophob substituierte Stärken, Alginate, Glucane, Chitine, Dextrane, Caseinate, Pektine, Proteine und Gummen, ferner Polyurethane, Polyacrylamide, Polyvinylalkohole, Polyacrylatem, wasserlösliche Silikonpolymere und dergleichen mehr.
Beispielsweise werden Cetylhydroxyethylcellulose, Hydroxypropylmethylcellulose und auch Polyethylenglycole mit langen PEG-einheiten, wie beispielsweise PEG-14 M, PEG-45 M von Amerchol und Oxethal VD 92 (Glyceryl-90 Isostearat + Laureth -2) von Zschimmer und Schwarz, verwendet

Weiterhin sind auch kationische Polymere als Verdicker bekannt, wie beispielsweise
1. Quaternäre Derivate von Celluloseethern, beispielsweise Polyquaternium 10, Polyquaternium 24
2. Quaternäre Derivate von Gummen, beispielsweise Guar Hydroxypropyltrimonium chlorid und Hydroxypropyltrimonium hydroxypropyl guar
3. Homopolymere und Copolymere von Dimethyl diallyammonium Chloride (DMDAAC), beispielsweise Polyquaternium 4 und 6 und 7
4. Homopolymere und Copolymere von Methacrylamidopropyl trimethyl ammonium chlorid (MAPTAC), beispielsweise Polyquaternium 28 und Polymethacrylamidopropyltrimonium chlorid
5. Homopolymere und Copolymere von Acrylamidopropyl trimethyl ammonium chlorid (APTAC)
6. Homopolymere und Copolymere von Methacryloyloxy ethyl trimethyl ammonium chlorid (METAC), beispielsweise Polyquaternium 32 und Polyquaternium 37
7. Homopolymere und Copolymere von Acryloyloxy ethyl trimethyl ammonium chlorid (AETAC), beispielsweise Polyquaternium 33
8. Homopolymere und Copolymere von Methacryloyloxy ethyl trimethyl ammonium methyl sulfat (METAMS), beispielsweise Polyquaternium 5 und Polyquaternium 11 und Polyquaternium 14 und Polyquaternium 15 und Polyquaternium 36 und Polyquaternium 45
9. Homopolymere und Copolymere von Vinylpyrrolidon, beispielsweise Luviquat 905
10. Kationische Polysaccharide basierend auf Hydroxyalkylcellulosen, Stärken, Hydroxyalkylstärken, Polymere basierend auf Arabinose Monomeren, Polymere abgeleitet von der Xylose, Fucose, Fructose, Galacturonsäure und Glucuronsäure, Galactosamin und Glucosamin, Acetylglucosamin, Galactose, Mannose. Beispielsweise Hydroxypropyl trimethyl ammoniumchlorid Derivate von Stärke, die von Korn, Kartoffel, Rice, Tapioaca, Weizen.
11. Ammonium substituierte Polydimethylsiloxane, beispielsweise Trimethylsilylamodimethicon (Dow Coming Q2-8220), Copolymere von Polydimethylsiloxan und Poly C2-C3 Alkylenether (Dow Corning 190, Dow Corning 2-5324, Dow Coming Q2-5220), Abil-Quat 3270
12. PVP
13. Polyethylenimine, beispielsweise Lupasol FG (BASF), Lupasol G-35 (BASF), Lupasol P (BASF)
14. Chitosan

Diese Vielzahl an Verdickungsmittel macht eine korrekte Auswahl für den Einsatz in tensidhaltigen Zubereitungen äußerst schwierig. Kosmetische oder dermatologische Zubereitungen, die sich somit verdicken lassen, zeigen zudem oftmals eine vom Verbraucher als unangenehm empfundene gelartig schleimige Konsistenz.

Eine vornehmliche Aufgabe der vorliegenden Erfindung ist es daher Verdickungsmittel bereit zu stellen, die bei der Einarbeitung in tensidhaltigen kosmetischen oder dermatologischen Zubereitungen zu einer vorteilhafte Viskosität der Zubereitungen führen.

Weiterer Nachteil bekannter Verdickungsmittel ist, dass sie in tensidhaltigen Zubereitungen häufig zu Trübungen und/oder Gelbfärbungen führen. Diesem Übelstande gilt es also, Abhilfe zu schaffen.

Die Produktion von kosmetischen Reinigungsmitteln zeigt seit Jahren eine steigende Tendenz. Dies ist vor allem auf das zunehmende Gesundheitsbewußtsein und Hygienebedürfnis der Verbraucher zurückzuführen.

Reinigung bedeutet das Entfernen von (Umwelt-) Schmutz und bewirkt damit eine Erhöhung des psychischen und physischen Wohlbefindens. Die Reinigung der Oberfläche von Haut und Haaren ist ein sehr komplexer, von vielen Parametern abhängiger Vorgang. Zum einen sollen von außen kommende Substanzen wie beispielsweise Kohlenwasserstoffe oder anorganische Pigmente aus unterschiedlichsten Umfeldern sowie Rückstände von Kosmetika oder auch unerwünschte Mikroorganismen möglichst vollständig entfernt werden. Zum anderen sind körpereigene Ausscheidungen wie Schweiß, Sebum, Haut- und Haarschuppen ohne tiefgreifende Eingriffe in das physiologische Gleichgewicht abzuwaschen.

Die Forderungen an die Eigenschaften kosmetischer Reinigungspräparate haben sich in den letzten Jahren stark gewandelt. Früher standen Effekte wie Reinigen und Schäumen im Vordergrund der Verbraucherwünsche. Zur Zeit sind die ökologischen, ökonomischen und insbesondere dermatologischen Eigenschaften der Produkte vorrangig, obwohl das Schaumvermögen nach wie vor eine entscheidende Rolle spielt, beispielsweise als Indikator, um Restmengen von Tensiden nach der Reinigung von Haut und Haaren zu entfernen oder um Überdosierungen bei der Anwendung zu vermeiden. Allerdings steht bei kosmetischen Produkten - im Gegensatz zu den meisten technischen Reinigungsmitteln - die Haut- und Schleimhautverträglichkeit absolut im Vordergrund; die Produkte sollen "mild" sein.

Kosmetische oder dermatologische Reinigungspräparate sind sogenannte "rinse off" Präparate, welche nach der Anwendung von der Haut abgespült werden. Sie werden in aller Regel in Form eines Schaums mit Wasser auf die zu reinigenden Körperpartien aufgetragen. Basis aller kosmetischen oder dermatologischen Reinigungspräparate sind waschaktive Tenside. Tenside sind amphiphile Stoffe, die organische, unpolare Substanzen in Wasser lösen können. Sie zeichnen sich durch ein ambivalentes Verhalten gegenüber Wasser und Lipiden aus: Das Tensidmolekül enthält mindestens je eine hydrophile und eine lipophile Gruppe, die die Anlagerung an der Grenzfläche zwischen diesen beiden Substanzklassen ermöglichen. Auf diese Weise sorgen Tenside für eine Herabsetzung der Oberflächenspannung des Wassers, die Benetzung der Haut, die Erleichterung der Schmutzentfernung und -lösung, ein leichtes Abspülen und - je nach Wunsch - auch für Schaumregulierung. Damit ist die Grundlage für die Schmutzentfernung lipidhaltiger Verschmutzungen gegeben.

Bei den hydrophilen Anteilen eines Tensidmoleküls handelt es sich meist um polare funktionelle Gruppen, beispielweise -COO⁻, -OSO₃²⁻, -SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wässriger Lösung bilden sie im sauren oder neutralen Milieu negativ geladene organische Ionen. Kationische Tenside sind beinahe ausschließlich durch das Vorhandensein einer quarternären Ammoniumgruppe gekennzeichnet. In wässriger Lösung bilden sie im sauren oder neutralen Milieu positiv geladene organische Ionen. Amphotere Tenside enthalten sowohl anionische als auch kationische Gruppen und verhalten sich demnach in wässriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

| | | |
|---|---|---|
| RNH₂⁺CH₂CH₂COOH X⁻ | (bei pH=2) | X⁻ = beliebiges Anion, z.B. Cl⁻ |
| RNH₂⁺CH₂CH₂COO⁻ | (bei pH=7) | |
| RNHCH₂CH₂COO⁻ B⁺ | (bei pH=12) | B⁺ = beliebiges Kation, z.B. Na⁺ |

Typisch für nicht-ionische Tenside sind Polyether-Ketten. Nicht-ionische Tenside bilden in wässrigem Medium keine Ionen.

WO 03013459 A1 und WO 03013467 A1 offenbaren Kombinationen aus Verdickungsmitteln für kosmetische oder dermatologische Zubereitungen umfassend ethoxilierte Fettsäure-Glycerinester und ethoxilierte Fettalkohole (PEG-90 Glyceryl Isostearate und Laureth-2) und als Co-Verdickungsmittel ethoxilierte Fettsäurepartialglyceride (PEG-100 Hydrogenated Glyceryl Palmate).
Als Tenside sind anionische bzw. amphotere Tenside, wie Natriumlaurethsulfat und Cocoamidopropylbetain sowie PEG-40 hydriertes Rizinusöl (nichtionisches Tensid) in den Beispielzubereitungen enthalten.

EP 1055407 A2 offenbart Haarbehandlungsmittel umfassend ethoxilierte Fettsäure-Glycerinester (Polyethylenglykol(7)-glycerylcocoat) und ethoxilierte Fettalkohole (Laureth-4 bzw. Laureth-23) und als Co-Verdickungsmittel ethoxilierte Fettsäurepartialglyceride (Polyethylenglykol(200)-glyceryltallowat).

WO 9963032 A1 offenbart Duschgele enthaltend eine Kombination aus Verdickungsmitteln umfassend ethoxilierte Fettsäure-Glycerinester, ethoxilierte Fettalkohole und nichtionische Tenside.

Viele tensidhaltige Formulierungen, insbesondere solche mit einem hohen Anteil an nichtionischen Tensiden, lassen sich nur unzureichend oder mitunter gar nicht verdicken. Diese Problem ist im sauren pH-Bereich stärker ausgeprägt als im neutralen bzw. alkalischen pH-Bereich. Systeme, die sich dennoch verdicken lassen zeigen dann allerdings, wie erwähnt, eine von den Verbrauchern als unangenehm empfundene gelartige, schleimige Konsistenz und bieten durch Trübungen und Gelbfärbungen eine unansehnliche Ästhetik.

Aufgabe der vorliegenden Erfindung ist es demnach kosmetische oder dermatologische Zubereitungen gemäß Anspruch 1, die einen Anteil nichtionischer Tenside aufweisen, bereit zu stellen, die diese Nachteile der bekannten tensidhaltigen Zubereitungen nicht aufweisen.

Es hat sich überraschend gezeigt, und darin liegt die Lösung dieser Aufgaben, dass eine Kombination verschiedener Verdickungsmittel entsprechend den Hauptansprüchen die Nachteile des Standes der Technik vermeiden helfen. Man unterscheidet erfindungsgemäß hierbei Verdickungsmittel und Co-Verdickungsmittel. Diese zeigen einzeln für die tensidhaltigen Zubereitungen keine oder eine nur unzureichende Verdickungswirkung. Zusammen zeigen sich jedoch überraschende Synergieeffekte.

Die innovative Auswahl und Kombination der erfindungsgemäßen Verdickungsmittel mit den Co-Verdickungsmittel aus der großen Anzahl möglicher Verdickungsmittel führt zu einer nicht vorhersehbaren vorteilhaften Einsetzbarkeit dieses Verdickungssystems in vielen kosmetischen und dermatologischen Formulierungen.

Die erfindungsgemäße Kombination besteht aus zwei Verdickungsmitteln (ethoxilierte Fettsäure-Glycerinester gemäss Formel (I) und ethoxilierte Fettalkohole) und mindestens einem Co-Verdickungsmittel (ethoxilierte Fettsäurepartialglyceride oder ethoxilierte Fettsäuremethylglykoside und/oder die Gemische beider Komponenten).

Zu den erfindungsgemäßen Verdickungsmitteln gehören ethoxilierte Fettsäure-Glycerinester gemäss Formel (I) und ethoxilierte Fettalkohole. Besonders bevorzugt sind Gemische mit einem Gehalt der Esterkomponente von 75% - 85 Gew.% bezogen auf die Gesamtverdickermenge.
Von den ethoxilierten Fettsäure-Glycerinestern sind Glycerinisostearatpolyglykolether der Formel (I):

C₁₇H₃₅COOCH₂COHHCH₂(OCH₂CH₂)ₙOH

n = 90
zu wählen
Von den ethoxilierten Fettalkoholen zeigen sich solche der Formel:

CₓH₂ₓ₊₁(OCH₂CH₂)ₙOH

X = 8 - 16, bevorzugt 12 - 14; n = 1 - 9, bevorzugt 2 - 4
als besonders geeignet.
Fettalkoholpolyglykolether mit einem durchschnittlichen Ethoxilierungsgrad von 2 mol Ethylenoxid sind besonders bevorzugt.

Bei den erfindungsgemäßen Verdickungsmitteln kann es sich beispielsweise um ein Gemisch, wie es unter dem Handlesnamen "Oxetal VD 92" und der INCI: PEG-90 Glyceryl Isostearate (and) Laureth-2 von der Fa. Zschimmer und Schwarz angeboten wird, handeln.

Zu den Co-Verdickungsmitteln gehören ethoxilierte Fettsäurepartialglyceride oder ethoxilierte Fettsäuremethylglykoside und/oder die Gemische beider Komponenten. Von den ethoxilierten Fettsäurepartialglyceriden zeigen sich insbesondere solche auf Basis von Palmöl mit folgender Fettsäurezusammensetzung als geeignet::
Ölsäure 37-50%
Palmitinsäure 35-45%
Linolsäure 9%
Stearinsäure 5%
Myristinsäure 1,5%
Besonders geeignet sind davon solche mit einem durchschnittlichen Ethoxilierungsgrad von 200 mol Ethylenoxid, wie sie z.B. unter dem Handelsnamen "Rewoderm LI 520-70" und der INCI: PEG-200 Hydrogenated Glyceryl Palmate von der Fa. Goldschmidt angeboten werden.
Von den ethoxilierten Fettsäuremethylglucosiden zeigen sich insbesondere die Diester der Ölsäure mit einem durchschnittlichen Ethoxilierungsgrad von 120 mol Ethylenoxid als geeignet, wie sie z.B. unter dem Handelsnamen "Glucamate DOE-120" und der INCI: PEG-120 Methyl Glucose Dioleate von der Fa. Amerchol angeboten werden.

Beispielsweise beträgt das Verhältnis Verdickungsmittel zu Co-Verdickungsmittel 80 : 20 bis 20 : 80, bevorzugt 70 : 30 bis 30 : 70, besonders bevorzugt 60 : 40 bis 40 : 60, in der erfindungsgemäßen Verdickungsmittelkombination.

Es hat sich überraschenderweise gezeigt, dass durch den Einsatz der Verdickungsmittel in Kombination mit einem Co-Verdickungsmittels in kosmetischen Zubereitungen, die jedes für sich alleine auf die in Frage kommenden Systeme keine oder eine nur unzureichende Verdickung zeigen, Synergieeffekte auftreten. Diese führen zu einem angenehm viskosen, jedoch gut fließfähigen und gut verteilbarem kosmetischen Produkt klarer Transparenz und ohne begleitende Gelbfärbung. Diese kosmetischen Zubereitungen können durch den Einsatz von Trübungs- oder Perlglanzmittel hinsichtlich ihrer Transparenz bis zur Lichtundurchlässigkeit eingestellt werden. Damit können auch kosmetische Produkte von cremeartigem Aussehen angeboten werden.

Da kosmetische Reinigungszubereitungen zumeist Tenside enthalten, ermöglicht der Einsatz der erfindungsgemäßen Verdickungsmittelkombination die Herstellung von kosmetischen Zubereitung, die einen hohen Anteil nichtionischer Tenside enthält und dies ohne Einbußen hinsichtlich Ästhetik oder Viskosität. Der Vorteil ist gerade im sauren pH-Bereich besonders ausgeprägt, denn eine Verdickung von tensidhaltigen Zubereitungen im sauren pH-Bereich ist durch die erfindungsgemäße Verdickungsmittelkombination möglich geworden. Der Einsatz führt sogar zu tensidhaltigen Zubereitungen, die für den Verbraucher angenehme Fließeigenschaften aufweisen. Der Einsatz nur einzelner aufgeführter Verdickungsmittel ergibt keine der vorteilhaften Eigenschaften, so dass die Innovation in der synergistischen Kombination der erfindungsgemäßen Verdickungsmittel mit den Co-Verdickungsmittel und deren Einsatz in kosmetischen Zubereitungen liegt.

Die tensidhaltige kosmetische Zubereitung enthält einen Anteil an nichtionischen Tensiden im Bereich von 40 bis 80 Gew.%, bezogen auf die insgesamt enthaltende Tensidmenge. Die Verdickung tensidhaltiger Zubereitungen mit einem Anteil, insbesondere mit einem hohen Anteil, nichtionischer Tenside durch die erfindungsgemäße Verdickungsmittelkombination führt zu angenehm viskosen, gut fließfähigen und gut auf der Haut und den Haaren verteilbaren Produkten mit klarer Transparenz. Dies ist insbesondere überraschend, da bekanntermaßen derartige Formulierungen in verdicktem Zustand üblicherweise gelartig sind und ein laminarer Fluss nicht mehr möglich ist.

Als besonders vorteilhaft haben sich tensidhaltige kosmetische Zubereitung erwiesen, die eine Kombination von anionischen und nichtionischen Tensiden bestehend aus
a. Alkylpolyglucosiden (APG) der Formel

   R-O-(CH₂- CH₂O)ₙ-Zₓ

   Alkyl(ether)sulfaten (AES) der Formel

   R-O--(CH₂- CH₂O)ₙSO₃M
b. PEG-Sorbitanester (PSE) der Formel 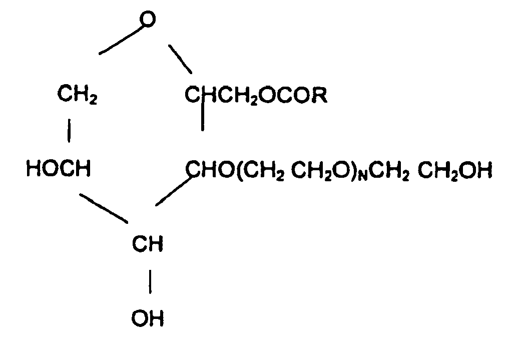 und
d. Alkylcitratsulfosuccinaten (ACS) der Formel 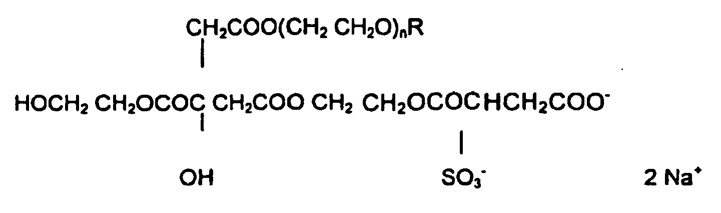 mit
   R = geradlinig oder verzweigte, gesättigte oder ungesättigte C₆-C₂₂ Kohlenwasserstoffkette; Z = Mono- oder Oligosaccharid; n = ganze Zahl von 0 - 10;
   x = ganze Zahl von 1 - 10; M = Alkali- oder Erdalkalimetall oder Ammonium;
   N = ganze Zahl von 0 - 60
   enthalten.

Es war für den Fachmann nicht vorauszusehen gewesen, dass diese erfindungsgemäßen Zubereitungen ein ausgezeichnetes Schaumverhalten, gute Reinigungsleistungen und vor allem gute Haut- und Haarverträglichkeit aufweisen. Insbesondere als Shampoo zeigten sich überraschend positive Eigenschaften, so wirkt das mit den erfindungsgemäßen Zubereitungen behandelte Haar äußerst gepflegt und lässt sich gut frisieren. Dies ist beispielsweise an der Kämmbarkeit, des Anscheins und der Fülle des Haares erkennbar.

Die erfindungsgemäße zusätzliche Kombination der anionischen und nichtionischen Tenside zeigt einen Synergismus, der für die überraschenderweise äußerst positiven Eigenschaften der kosmetischen Zubereitung verantwortlich ist.

In den aufgeführten Tensiden wird R in der Strukturformel vorteilhaft gewählt aus der Gruppe der unverzweigten Alkylreste, wobei der Hexyl-, der Heptyl-, der Octyl- der Nonyl- der Decyl-, der Undecyl-, der Dodecyl-, und der Tetradecylrest bevorzugt werden.

Die Gesamtmenge der anionischen und nichtionischen Tenside beträgt 8 - 15 Gew.%, insbesondere 10 - 13 Gew.%, bezogen auf die Gesamtzubereitung.

Das Tensidverhältnis der anionischenTenside (AES, ACS) zu den nichtionischen Tensiden (APG, PSE) beträgt vorteilhafterweise 20 : 80 bis 60 : 40, insbesondere 40 : 60 bis 55 : 45. Weiterhin kann das Verhältnis von AES zu ACS 20 : 80 bis 60 : 40, insbesondere 40 : 60 bis 55 : 45 und/oder das Verhältnis von APG zu PSE 90 : 10 bis 50 : 50, insbesondere 80 : 20 bis 60 : 40 gewählt werden. Durch diese innovative Tensidkombination und Mengenverhältniswahl resultieren die angesprochenen positiven Eigenschaften der erfindungsgemäßen Zubereitungen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Farbstoffe, Pigmente, die eine färbende Wirkung haben, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist in kosmetischen und/oder dermatologischen Zubereitungen im allgemeinen bevorzugt. Erfindungsgemäß können als günstige Antioxidantien alle für kosmetische und/oder dermatologische Anwendungen geeigneten oder gebräuchlichen Antioxidantien verwendet werden.
Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Camosin und deren Derivate (z.B. Anserin), Carotinoide, Carotine (z.B. α-Carotin, β-carotin, ψ-Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.%, besonders bevorzugt 0,05 - 20 Gew.-%, insbesondere 1 - 10 Gew.%, bezogen auf das Gesamtgewicht der Zubereitung.

Sofern Vitamin E und/oder dessen Derivate das oder die Antioxidantien darstellen, ist vorteilhaft deren jeweilige Konzentrationen aus dem Bereich von 0,001 - 10 Gew.%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft deren jeweilige Konzentrationen aus dem Bereich von 0,001 - 10 Gew.-%, bezogen auf das Gesamtgewicht der Formulierung, zu wählen.

Die vorliegende Erfindung betrifft insbesondere waschaktive haarkosmetische Zubereitungen, landläufig als Shampoos bezeichnet. Insbesondere betrifft die vorliegende Erfindung haarkosmetische Wirkstoffkombinationen und Zubereitungen zur Pflege des Haars und der Kopfhaut.

Weiterhin betrifft die Erfindung Duschgele, Cleansing-Zubereitungen, Handwaschprodukte, Badezubereitungen, Make-up-Entferner und/oder Rasierprodukte. Die Produkte können niedrig- oder hochviskos sein, leicht oder stark schäumen und/oder als antibakterielle rinse-off Formulierungen eingesetzt werden.
Zubereitungen dieser Art sind beispielsweise als Schaum- und Duschbäder, flüssige Seifen , Intimwaschmittel, spezielle Reinigungsmittel für Kleinkinder und dergleichen bekannt.

Die Herstellung der erfindungsgemäßen kosmetischen und/oder dermatologischen Zubereitungen erfolgt in der dem Fachmann bekannten üblichen Weise, zumeist dergestalt, dass die erfindungsgemäß verwendeten Substanzen oder Vorlösung dieser Substanzen bei gleichmäßigem Rühren und ggf. unter Erwärmen gelöst bzw. dispergiert werden.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele 1 - 3

### Conditioner-Shampoo mit Perlglanz

| | **1** | **2** | **3** |
|---|---|---|---|
| Polymer JR 400¹ | 0,5 | 0,5 | 0,5 |
| Texapon K 14 S spezial² | 4,0 | 5,0 | 4,5 |
| Plantacare 2000³ | 9,0 | 8,0 | 8,5 |
| Perlglanzmittel | 2,0 | 2,0 | 2,0 |
| Rewopol SBCS 50⁴ | 6,0 | 5,5 | 6,2 |
| Atlas G-4280⁵ | 3,2 | 3,5 | 3,0 |
| Oxetal VD 92⁶ | 3,0 | 1,2 | 2,8 |
| Rewoderm LI 520-70⁷ | 4,0 | 3,0 | 4,2 |
| Konservierungsmittel, Parfüm, pH-Einstellung und Lösungsvermittler | q.s. | q.s. | q.s. |
| Wasser, VES (vollentsalzt) | ad 100,0 | ad 100,0 | ad 100,0 |

Der pH-Wert wird auf 5,5 eingestellt.

### Beispiele 4 - 6

### klares Conditioner-Shampoo

| | **4** | **5** | **6** |
|---|---|---|---|
| Polymer JR 400¹ | 0,5 | 0,5 | 0,5 |
| Texapon K 14 S spezial² | 4,2 | 5,0 | 4,8 |
| Plantacare 2000³ | 8,8 | 8,0 | 8,3 |
| Rewopol SBCS 50⁴ | 6,2 | 5,7 | 6,3 |
| Atlas G-4280⁵ | 3,0 | 3,5 | 3,1 |
| Oxetal VD 92⁶ | 3,2 | 1,0 | 3,0 |
| Rewoderm LI 520-70⁷ | 4,0 | 3,2 | 4,0 |
| Komplexmittel, Konservierungsmittel, Parfüm, pH-Einstellung und Lösungsvermittler | q.s. | q.s. | q.s. |
| Wasser, VES | ad 100,0 | ad 100,0 | ad 100,0 |

Der pH-Wert wird auf 5,5 eingestellt.

### Beispiele 7 - 9

### klares Light-Shampoo mit Volumeneffekt

| | **7** | **8** | **9** |
|---|---|---|---|
| Texapon K 14 S Spezial² | 4,0 | 5,0 | 4,5 |
| Plantacare 2000³ | 9,2 | 8,0 | 8,4 |
| Rewopol SBCS 50⁴ | 6,0 | 5,5 | 6,2 |
| Atlas G-4280⁵ | 3,0 | 3,5 | 3,2 |
| Oxetal VD 92⁶ | 3,0 | 1,5 | 3,0 |
| Rewoderm LI 520-70⁷ | 4,0 | 3,2 | 4,2 |
| Komplexbildner, Konservierungsmittel, Parfüm, pH-Einstellung und Lösungsvermittler | q.s. | q.s. | q.s. |
| Wasser, VES | ad 100,0 | ad 100,0 | ad 100,0 |

Der pH-Wert wird auf 5,5 eingestellt.

Es bedeuten:
¹kationisches Cellulosederivat der Fa. Amerchol,
²Myrethsulfat 70%ig der Fa. Cognis (AES)
³ Decylgucoside 50%ig der Fa. Cognis (APG)
⁴Citronensäurealkylpolyglykolester-sulfosuccinat, Dinatriumsalz 38%ig der Fa. Goldschmidt (ACS) ⁵PEG-80 Sorbitan Laurate 73%ig der Fa. Uniqema (PSE)
⁶Glycerinisostearatpolyglykolether / Fettalkoholpolyglykolether 77%ig der Fa. Zschimmer & Schwarz
⁷Palmölfettsäure-partialglycerid-polyglykolether 70%ig der Fa. Goldschmidt

## Patentansprüche

1. Kosmetische Zubereitung umfassend eine Kombination aus Verdickungsmitteln umfassend ethoxilierte Fettsäure-Glycerinester und ethoxilierte Fettalkohole und mindestens einem von den Verdickungsmitteln verschiedenem Co-Verdickungsmittel gewählt aus ethoxillerten Fettsäurepartialglyceriden oder ethoxilierten Fettsäuremethylglykosiden und/oder deren Gemische, **dadurch gekennzeichnet, dass** die Zubereitung nichtionische Tenside enthalt, wobei der Anteil nichtionischer Tenside im Bereich von 40 bis 80 Gew.%, bezogen auf die insgesamt enthaltende Tensidmenge, beträgt und dass aus den ethoxilierten Fettsäure-Glycerinestern die Glycerinisostearatpolyglykolether der Formel
C₁₇H₃₅COOCH₂COHHCH₂(OCH₂CH₂)nOH
mit n = 90 gewählt werden.

2. Zubereitung nach Anspruch 1, **dadurch gekennzeichnet, dass** in der Kombination die Esterkomponente des Verdickungsmittels mit einem Gehalt von 75% - 85 Gew.% bezogen auf die Gesamtverdickermenge, ohne Co-Verdicker, enthalten ist.

3. Zubereitung nach Anspruch 1 oder 2, **dadurch gekennzeichnet, dass** aus den ethoxilierten Fettalkoholen die Fettalkoholpolyglykolether der Formel
CₓH₂ₓ₊₁(OCH₂CH₂)ₙOH
mit x=12 bis 14, n= 2 - 4, und
von den Co-Verdickungsmitteln aus den ethoxilierten Fettsäurepartialglyceriden solche auf Basis von Palmöl der Fettsäurezusammensetzung
| | |
|---|---|
| Ölsäure | 37-50 Gew.%, |
| Palmitinsäure | 35-45 Gew.%, |
| Linolsäure | 9 Gew.%, |
| Stearinsäure | 5 Gew.%, |
| Myristinsäure | 1,5 Gew.%, bezogen auf die Fettsäurezusammensetzung, |
mit einem Ethoxilierungsgrad von 200 mol Ethylenoxid oder aus den ethoxilierten Fettsäuremethylglykosiden die Diester der Ölsäure mit einem durchschnittlichen Ethoxilierungsgrad von 120 mol Ethylenoxid und/oder deren Gemische gewählt werden.

4. Zubereitung nach Anspruch 1, 2 oder 3, **dadurch gekennzeichnet, dass** als Fettalkoholpolyglykolether solche mit einem durchschnittlichen Ethoxilierungsgrad von 2 mol Ethylenoxid ausgewählt werden.

5. Zubereitung nach einem der Ansprüche 1 bis 4, **dadurch gekennzeichnet, dass** das Verhältnis Verdickungsmittel zu Co-Verdickungsmittel 80 : 20 bis 20 : 80, bevorzugt 70 : 30 bis 30 : 70, besonders bevorzugt 60 : 40 bis 40 : 60, beträgt.

6. Kosmetische Zubereitung nach Anspruch 1 enthaltend eine Kombination von anionischen und nichtionischen Tensiden bestehend aus
a. Alkylpolyglucosiden (APG) der Formel
R-O-(CH₂- CH₂O)ₙ-Zₓ
b. Alkyl(ether)sulfaten (AES) der Formel
R-O-(CH₂- CH₂O)ₙSO₃M
a. PEG-Sorbitanester (PSE) der Formel 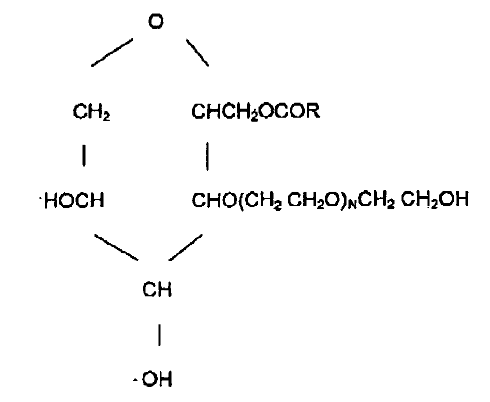 und
d. Alkylcitratsulfosuccinaten (ACS) der Formel 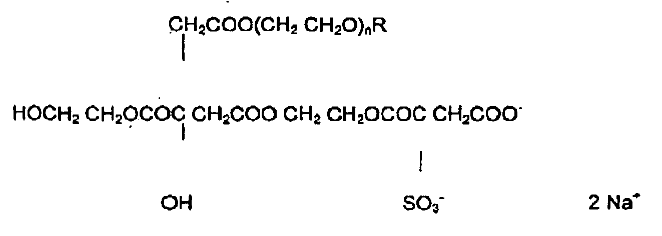 mit
R = geradlinig oder verzweigte, gesättigte oder ungesättigte C6-C22 Kohlenwasserstoffkette; Z = Mono- oder Oligosaccharid; n = ganze Zahl von 0-10;
x = ganze Zahl von 1 - 10; M = Alkali- oder Erdalkalimetall oder Ammonium;
N = ganze Zahl von 0 - 60

7. Kosmetisch Zubereitung nach Anspruch 6, **dadurch gekennzeichnet, dass** R in der Strukturformel gewählt wird aus der Gruppe der unverzweigten Alkylreste, Insbesondere aus den Hexyl-, Heptyl-, Octyl-, Nonyl-, Decyl-, Undecyl-, Dodecyl- und/oder den Tetradecylresten.

8. Kosmetische Zubereitung nach Anspruch 6 oder 7, **dadurch gekennzeichnet, dass** das Tensidverhältnis anionischesTensid (AES, ACS) zu nichtionisches Tensid (APG, PSE) 20 : 80 bis 60 : 40, insbesondere 40 : 60 bis 55 : 45 beträgt.

9. Kosmetische Zubereitung nach Anspruch 6, 7 oder 8, **dadurch gekennzeichnet, dass** das Verhältnis von AES : ACS 20 : 80 bis 60 : 40, insbesondere 40 : 60 bis 55 : 45 und/oder das Verhältnis von APG : PSE 90 : 10 bis 50 : 50, insbesondere 80 : 20 bis 60 : 40 beträgt.

10. Kosmetische Zubereitung nach einem der Ansprüche 6 bis 9, **dadurch gekennzeichnet, dass** die Gesamtmenge der Tenside 8 - 15 Gew.%, insbesondere 10 -13 Gew.%, bezogen auf die Gesamtzubereitung beträgt.

11. Haarshampoo enthaltend eine Zubereitung nach einem der Ansprüche 1 bis 10.

12. Verwendung der Zubereitungen nach einem der Ansprüchen 1 bis 10 als Reinigungsmittel für Haut und/oder Haare, insbesondere als Haarshampoo.

13. Verwendung der Zubereitungen nach einem der Ansprüche 1 bis 10 als Duschgel, Cleansing-Zubereitung, Handwaschprodukt, Badezubereitung, Make-up-Entferner. Intimwaschmittel, spezielle Reinigungsmittel für Kleinkinder und/oder Rasierprodukt.

## Claims

1. Cosmetic preparation comprising a combination of thickeners comprising ethoxylated fatty acid glycerol esters and ethoxylated fatty alcohols and at least one co-thickener different from the thickeners and selected from ethoxylated fatty acid partial glycerides or ethoxylated fatty acid methyl glycosides and/or mixtures thereof, **characterized in that** the preparation comprises nonionic surfactants, where the fraction of nonionic surfactants is in the range from 40 to 80% by weight, based on the amount of surfactant present overall, and **in that** from the ethoxylated fatty acid glycerol esters the glycerol isostearate polyglycol ethers, of the formula
C₁₇H₃₅COOCH₂COHHCH₂ (OCH₂CH₂) nOH
where n = 90, are selected.

2. Preparation according to Claim 1, **characterized in that**, in the combination, the ester component of the thickener is present with a content of 75%-85% by weight, based on the total amount of thickener, without co-thickener.

3. Preparation according to Claim 1 or 2, **characterized in that**
from the ethoxylated fatty alcohols the fatty alcohol polyglycol ethers of the formula
CₓH₂ₓ₊₁(OCH₂CH₂)ₙOH
where x = 12 to 14, n = 2 to 4, are selected and
of the co-thickeners from the ethoxylated fatty acid partial glycerides those based on palm oil of the fatty acid composition
| | |
|---|---|
| oleic acid | 37-50% by weight |
| palmitic acid | 35-45% by weight, |
| linoleic acid | 9% by weight, |
| stearic acid | 5% by weight, |
| myristic acid | 1.5% by weight, based on the fatty acid composition, |
with a degree of ethoxylation of 200 mol of ethylene oxide are selected, or from the ethoxylated fatty acid methyl glycosides the diesters of oleic acid with an average degree of ethoxylation of 120 mol of ethylene oxide and/or mixtures thereof are selected.

4. Preparation according to Claims 1, 2 or 3, **characterized in that** the fatty alcohol polyglycol ethers selected are those with an average degree of ethoxylation of 2 mol of ethylene oxide.

5. Preparation according to one of Claims 1 to 4, **characterized in that** the ratio of thickener to co-thickener is 80:20 to 20:80, preferably 70:30 to 30:70, particularly preferably 60:40 to 40:60.

6. Cosmetic preparation according to Claim 1, comprising a combination of anionic and nonionic surfactants consisting of
a. alkyl polyglucosides (APG) of the formula
R-O- (CH₂-CH₂O)ₙ-Zₓ
b. alkyl (ether) sulphate (AES) of the formula
R-O- (CH₂-CH₂O)ₙSO₃M
c. PEG sorbitan esters (PSE) of the formula 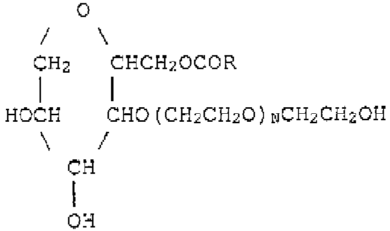 and
d. alkyl citrate sulphosuccinates (ACS) of the formula 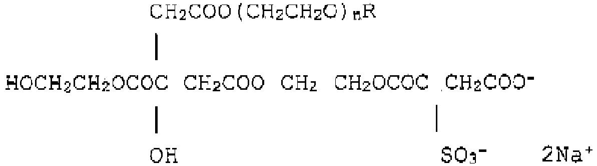 where
R = straight-line or branched, saturated or unsaturated
C6-C22 hydrocarbon chain; Z = mono- or oligosaccharide;
n = integer from 0-10; x = integer from 1-10;
M = alkali metal or alkaline earth metal or ammonium;
N = integer from 0-60.

7. Cosmetic preparation according to Claim 6, **characterized in that** R in the structural formula is selected from the group of unbranched alkyl radicals, in particular from the hexyl, heptyl, octyl, nonyl, decyl, undecyl, dodecyl and/or the tetradecyl radicals.

8. Cosmetic preparation according to Claim 6 or 7, **characterized in that** the surfactant ratio of anionic surfactant (AES, ACS) to nonionic surfactant (APG, PSE) is 20:80 to 60:40, in particular 40:50 to 55:45.

9. Cosmetic preparation according to Claims. 6, 7 or 8, **characterized in that** the ratio of AES:ACS is 20:80 to 60:40, in particular 40:60 to 55:45 and/or the ratio of APG:PSE is 90:10 to 50:50, in particular 80:20 to 50:40.

10. Cosmetic preparation according to one of Claims 6 to 9, **characterized in that** the total amount of the surfactants is 8-15% by weight, in particular 10-13% by weight, based on the total preparation.

11. Hair shampoo comprising a preparation according to one of Claims 1 to 10.

12. Use of the preparations according to one of Claims 1 to 10 as cleansing composition for skin and/or hair, in particular as hair shampoo.

13. Use of the preparations according to one of Claims 1 to 10 as shower gel, cleansing preparation, handwashing product, bath preparation, make-up remover, intimate washing composition, special cleaning compositions for small children and/or shaving product.

## Revendications

1. Préparation cosmétique comprenant une association d'épaississants comprenant des esters de glycérol-acides gras éthoxylés et des alcools gras éthoxylés et au moins un co-épaississant différent des épaississants, choisi parmi des glycérides partiels diacides gras éthoxylés ou des méthylglycosides d'acides gras éthoxylés et/ou des mélanges de ceux-ci, **caractérisée en ce que** la préparation contient des tensioactifs non ioniques, la proportion de tensioactifs non ioniques se situant dans la plage de 40 à 80 % en poids, par rapport à la quantité totale de tensioactifs contenue, et **en ce qu'**on choisit parmi les esters de glycérol-acides gras éthoxylés, les polyglycoléthers d'isostéarate de glycérol de formule
C₁₇H₃₅COOCH₂COHHCH₂ (OCH₂CH₂)ₙOH
où n = 90

2. Préparation selon la revendication 1, **caractérisée en ce que** dans l'association le composant ester de l'épaississant est contenu en une proportion de 75 % - 85 % en poids par rapport à la quantité totale d'épaississants, sans co-épaississants.

3. Préparation selon la revendication 1 ou 2, **caractérisée en ce qu'**on choisit
parmi les alcools gras éthoxylés, les polyglycoléthers d'alcools gras de formule
CₓH₂ₓ₊₁(OCH₂CH₂)ₙOH où x = 12 à 14, n = 2 à 4, et
parmi les co-épaississants du groupe des glycérides partiels d'acides gras éthoxylés, ceux à base d'huile de palme ayant la composition en acides gras
| | |
|---|---|
| acide oléique | 37-50 % en poids, |
| acide palmitique | 35-45 % en poids, |
| acide linoléique | 9 % en poids, |
| acide stérique | 5 % en poids, |
| acide myristique | 1,5 % en poids, par rapport à la composition d'acides gras, |
ayant un degré d'éthoxylation de 200 moles d'oxyde d'éthylène ou, dans le groupe des méthylglycosides d'acides gras éthoxylés, les diesters de l'acide oléique ayant un degré d'éthoxylation moyen de 120 moles d'oxyde d'éthylène et/ou des mélanges de ceux-ci.

4. Préparation selon la revendication 1, 2 ou 3, **caractérisée en ce qu'**on choisit comme polyglycoléthers d'acides gras ceux ayant un degré d'éthoxylation moyen de 2 moles d'oxyde d'éthylène.

5. Préparation selon l'une quelconque des revendications 1 à 4, **caractérisée en ce que** le rapport des épaississants au(x) co-épaississant (s) vaut de 80 : 20 à 20 : 80, de préférence de 70 : 30 à 30 : 70, de façon particulièrement préférée de 60 : 40 à 40 : 60.

6. Préparation cosmétique selon la revendication 1, contenant une association de tensioactifs anioniques et de tensioactifs non ioniques, constituée
a. d'alkylpolyglucosides (APG) de formule
R-O-(CH₂-CH₂O)ₙ-Zₓ
b. d'alkyl(éther)sulfates (AES) de formule
R-O-(CH₂-CH₂O)ₙSO₃M
c. de PEG-esters de sorbitanne (PSE) de formule 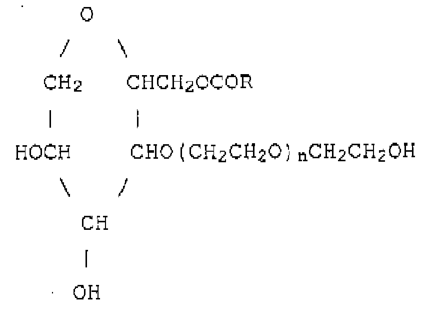 et
d. d'alkylcitratesulfosuccinates (ACS) de formule 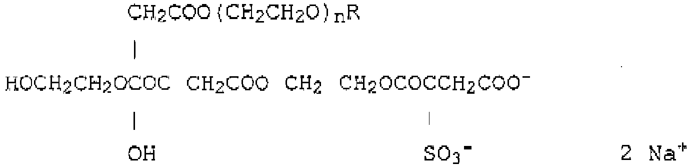 où
R représente une chaîne hydrocarbonée en C₆-C₂₂ droite ou ramifiée,, saturée ou insaturée ; Z représente un mono- ou oligosaccharide ; n est un nombre entier valant de 0 à 10 ; x est un nombre entier valant de 1 à 10 ; M représente un métal alcalin ou alcalino-terreux ou l'ammonium ; N est un nombre entier valant de 0 à 60.

7. Préparation cosmétique selon la revendication 6, **caractérisée en ce que** dans la formule développée R est choisi dans le groupe des radicaux alkyle non ramifiés, en particulier des radicaux hexyle, heptyle, octyle, nonyle, décyle, undécyle, dodécyle et/ou tétradécyle.

8. Préparation cosmétique selon la revendication 6 ou 7, **caractérisée en ce que** le rapport de tensioactifs du tensioactif anionique (AES, ACS) au tensioactif non ionique (APG, PSE) vaut de 20 : 80 à 60 : 40, en particulier de 40 : 60 à 55 : 45.

9. Préparation cosmétique selon la revendication 6, 7 ou 8, **caractérisée en ce que** le rapport de AES à ACS vaut de 20 : 80 à 60 : 40, en particulier de 40 : 60 à 55 : 45 et/ou le rapport de APG à PSE vaut de 90 : 10 à 50 : 50, en particulier de 80 : 20 à 60 : 40.

10. Préparation cosmétique selon l'une quelconque des revendications 6 à 9, **caractérisée en ce que** la quantité totale des tensioactifs vaut de 8 à 15 % en poids, en particulier de 10 à 13 % en poids, par rapport à la préparation totale.

11. Shampooing capillaire, contenant une préparation selon l'une quelconque des revendications 1 à 10.

12. Utilisation des préparations selon l'une quelconque des revendications 1 à 10, en tant que produit de nettoyage pour la peau et/ou les cheveux, en particulier en tant que shampooing capillaire.

13. Utilisation des préparations selon l'une quelconque des revendications 1 à 10, en tant que gel douche, préparation de nettoyage, produit pour le lavage des mains, préparation pour le bain, démaquillant, produit de lavage intime, produit de nettoyage spécial pour jeunes enfants et/ou produit de rasage.
